# EUROPÄISCHE PATENTSCHRIFT

(11) **EP 3 479 855 B1**
(45) Veröffentlichungstag und Bekanntmachung des Hinweises auf die Patenterteilung: **17.03.2021**
(21) Anmeldenummer: 18204411.5
(22) Anmeldetag: 05.11.2018
(51) Int. Cl.: A61M 1/16

(54) **VORRICHTUNG ZUR EXTRAKORPORALEN BLUTBEHANDLUNG MIT GRAVIMETRISCHER BILANZIERUNG UND MÖGLICHKEIT ZUR ULTRAFILTRATION**
DEVICE FOR EXTRACORPOREAL BLOOD TREATMENT WITH GRAVIMETRIC BALANCING AND ULTRAFILTRATION
DISPOSITIF DE TRAITEMENT DU SANG EXTRACORPOREL À COMPTABILISATION GRAVIMÉTRIQUE ET À POSSIBILITÉ D'ULTRAFILTRATION

(30) Priorität: 07.11.2017 DE 102017125962
(43) Veröffentlichungstag der Anmeldung: 08.05.2019
(73) Patentinhaber: B. Braun Avitum AG, 34212 Melsungen (DE)
(72) Erfinder: STEGER, Jennifer, 34212 Melsungen (DE); WEISER, Markus, 2384 Breitenfurt (AT)
(74) Vertreter: Winter, Brandl, Fürniss, Hübner, Röss, Kaiser, Polte - Partnerschaft mbB

(56) Entgegenhaltungen:
- EP-A1- 1 590 017
- EP-A2- 1 684 825
- US-A- 5 344 568
- US-A1- 2013 012 914
- US-A1- 2015 060 362

## Beschreibung

Die Erfindung betrifft eine Vorrichtung zur extrakorporalen Blutbehandlung, insbesondere eine Dialysemaschine, mit einer Internfluidik, an die eine Blutbehandlungseinheit, insbesondere ein Dialysator, anschließbar ist, wobei die Internfluidik zumindest eine frischstromseitige Bilanzierungskammer zur Bilanzierung von zur Blutbehandlungseinheit fließender Frischbehandlungsflüssigkeit und zumindest eine brauchstromseitige Bilanzierungskammer zur Bilanzierung von von der Blutbehandlungseinheit abströmender Brauchbehandlungsflüssigkeit umfasst.

In der Dialysetechnik ist es essenziell, die Dialysierflüssigkeit auf ihrem Weg zum Dialysator und vom Dialysator zum Abfluss zu bilanzieren. Daher werden bei heutigen Dialyseanwendung Dialysemaschinen genutzt, die Bilanzierungseinrichtungen umfassen. Diese überwachen und stellen sicher, dass ein dem Dialysator über einen bestimmten Zeitraum zugeführtes Flüssigkeitsvolumen einem dem Dialysator über den gleichen Zeitraum entnommenem Flüssigkeitsvolumen entspricht, die beiden Volumina also exakt identisch sind. Bei einigen Anwendungen kann eine Ultrafiltration, also ein Flüssigkeitsentzug aus dem Patienten, erwünscht sein. In einem solchen Fall muss auch die Ultrafiltration exakt bilanziert werden, so dass dem Patienten über die Dauer einer Therapie ein genau definiertes Ultrafiltrationsvolumen abgenommen wird. Zusammenfassend kann man also sagen, dass mittels einer Bilanzierung überwacht wird, dass dem Patienten keine Flüssigkeit oder nur genau eine beabsichtigte Flüssigkeitsmenge entzogen wird.

Bei bekannten Dialysemaschinen und Verfahren wird ein dem Patienten entzogenes Ultrafiltrationsvolumen von der Bilanzierungseinrichtung nicht direkt erfasst. In der Praxis kommt es vor allem bei Bilanzkammersystemen mit Membranen zu Fehlbilanzierungen durch das System, die zum Beispiel durch variierenden Druck, variierende Temperatur und variierende Volumenstromraten innerhalb des Bilanzierungssystems hervorgerufen werden. Bilanzierungsfehler können beispielsweise zu einem unerwünschten (zusätzlichen) Flüssigkeitsentzug durch die Bilanzkammer führen. Insbesondere durch Änderung der Gegendrücke, die auf die Bilanzierungskammern wirken, kann es im Rahmen einer Dialysebehandlung bei bekannten System und Verfahren zu Fehlbilanzen und zu Abweichungen in der (tatsächlichen) Flüssigkeitsbilanz kommen.

Zur Bilanzierung von Dialysierflüssigkeit im Rahmen von extrakorporalen Blutbehandlungen (und ggf. eines Ultrafiltrationsvolumens) sind unterschiedliche Verfahren sowie Vorrichtungen Stand der Technik. Bekannt sind zum Beispiel Flusssensoren (siehe beispielsweise WO 2013/164089), Massedurchflussmesser unter Nutzung des Coriolis-Effekts, Duplexpumpentechnik, Bilanzkammersysteme oder gravimetrische Bilanzierungssysteme in Form von Beutelwaagen (siehe zum Beispiel DE 4122171 (A1) oder EP 611228 B1). Außerdem sind aus dem Stand der Technik Bilanzierungsverfahren ohne Bilanzkammern mit Membranen bekannt, zum Beispiel mit Coriolis Messzellen oder Bilanzierungsverfahren mit hochgenauen, wechselseitigen Pumpen. Stand der Technik ist auch aus den Druckschriften US 2013 012914 A1, US 2015 060362 A1, US 5 344 568 A1, EP 1 684 825 A2 und EP 1 590 017 A1 bekannt.

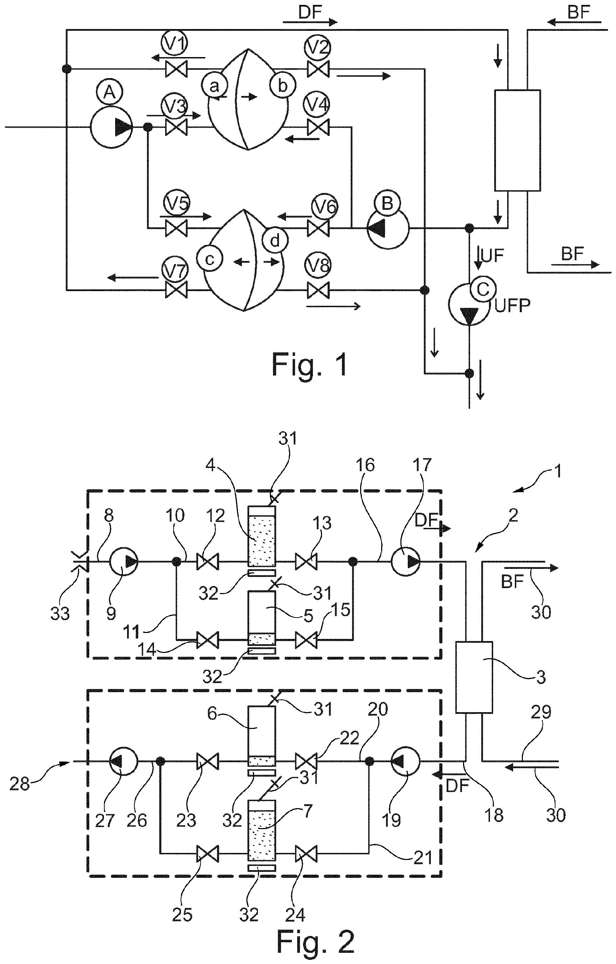

Ein bekanntes Bilanzkammersystem (siehe auch Abbildung in Figur 1), bei dem eine Bilanzierung der durch das System geförderten Behandlungsflüssigkeit volumetrisch erfolgt, besteht aus zwei Bilanzkammereinheiten, die durch jeweils eine bewegliche Membran in insgesamt vier Kammern (a, b, c, d) unterteilt sind. Zwei Pumpen (A, B) dienen dem Füllen der Kammern a und c bzw. b und d. Mittels einer weiteren Pumpe C wird eine definierte Flüssigkeitsentzugsrate (Ultrafiltrationsrate) erzwungen. Die Funktion des Systems ist in zwei Phasen unterteilt.

In einer ersten Phase liegen die folgenden Startbedingung vor: Kammer a leer, Kammer b voll (die Membran befindet sich in Figur 1 ganz links), Kammer c voll, Kammer d leer (die Membran befindet ist in Figur 1 ganz rechts). Die Ventile sind wie folgt geschaltet: V1 geschlossen, V2 offen, V3 offen, V4 geschlossen, V5 geschlossen, V6 offen, V7 offen, V8 geschlossen. Die Pumpe A füllt somit Kammer a durch V3 mit frischer Dialysierflüssigkeit. Dadurch wird die verbrauchte Dialysierflüssigkeit, die sich in Kammer b befindet, durch V2 in den Abfluss geleitet. Somit füllt Pumpe B Kammer d durch V6 mit verbrauchter Dialysierflüssigkeit. Dadurch wird die frische Dialysierflüssigkeit, die sich in Kammer c befindet, durch V7 zum Dialysator gefördert.

In einer zweiten Phase liegen die folgenden Startbedingungen vor: Kammer b leer, Kammer a voll (die Membran befindet sich in Figur 1 ganz rechts), Kammer d voll, Kammer c leer (die Membran befindet sich in Figur 1 ganz links). Die Ventile sind wie folgt geschaltet: V1 offen, V2 geschlossen, V3 geschlossen, V4 offen, V5 offen, V6 geschlossen, V7 geschlossen, V8 offen. Die Pumpe A füllt somit Kammer c durch V5 mit frischer Dialysierflüssigkeit. Dadurch wird die verbrauchte Dialysierflüssigkeit, die sich in Kammer d befindet, durch V8 in den Abfluss geleitet. Somit füllt Pumpe B Kammer b durch V4 mit verbrauchter Dialysierflüssigkeit. Dadurch wird die frische Dialysierflüssigkeit, die sich in Kammer a befindet, durch V1 zum Dialysator gefördert.

Zur Bestimmung der Exaktheit der Flüssigkeitsbilanz, insbesondere des Flüssigkeitsentzugs, wird der Patient vor sowie nach Abschluss der Therapie gewogen und aus Gewichtsänderungen Rückschluss auf seinen Flüssigkeitshaushalt gezogen. Im Hinblick auf den Bilanzabgleich ist bei dem obigen bekannten Verfahren und Vorrichtung von Nachteil, dass aufgrund der spezifischen Dichte ein Liter verbrauchter Dialysierflüssigkeit nicht ein Gewicht von einem Kilogramm aufweist. Erschwerend kommt hinzu, dass die spezifische Dichte von Dialysierflüssigkeit von ihrer exakten Zusammensetzung abhängig ist, demnach verbrauchte Dialysierflüssigkeit eine andere Dichte als frische Dialysierflüssigkeit besitzt. Außerdem ist die Dichte verbrauchter Dialysierflüssigkeit patientenspezifisch und ändert sich mit fortschreitender Reinigung des Blutes. Bei bekannten volumetrischen Verfahren wird dennoch weitestgehend angenommen, dass ein Kilogramm gebrachter Behandlungsflüssigkeit einem Volumen von etwa 1000 ml entspricht, wodurch es unweigerlich zu Ultrafiltrationsabweichungen kommt. Eine Bilanzierung anhand einer Abstimmung geförderter / bilanzierter Volumina mit Gewichtsänderungen des Patienten ist daher grundlegend problematisch.

Ein weiterer Nachteil ist, dass ein volumetrisches Verfahren in der Regel sensibel gegenüber Druck- und/oder Temperaturänderungen der zu bilanzierenden Behandlungsflüssigkeit ist:
Zum Beispiel kühlt die Behandlungsflüssigkeit bei einem vorstehend beschriebenen System auf dem Weg von der Bilanzkammereingangsseite zur Bilanzkammerausgangseite um einige Grad Celsius ab. Dadurch nimmt die Dichte der Behandlungsflüssigkeit zu und es kommt zu Bilanzierungsfehlern. Zwar ist eine Ultrafiltration zunächst abhängig von der Leistung der Ultrafiltrationspumpe, allerdings können insbesondere Bilanzierungsfehler aufgrund von an der Bilanzkammer vorliegenden Temperaturverhältnissen, insbesondere von Temperaturdifferenzen zwischen Bilanzierungskammer-Eingang und Bilanzierungskammer-Ausgang zu einem unerwünschten (zusätzlichen) Flüssigkeitsentzug durch die Bilanzkammern führen. Dort oder an anderer Stelle vorliegende Temperaturänderungen oder -differenzen ziehen eine Änderung des Volumens der bilanzierten Flüssigkeit mit sich, die durch Änderung der Dichte der Flüssigkeit in Abhängigkeit der Temperatur bedingt ist, und führen somit zu einer Fehlbilanz.

Außerdem unterscheiden sich in der Regel die Drücke auf der Bilanzkammereingangsseite und der Bilanzkammerausgangsseite. Ein erhöhter Druck führt dazu, dass sich die Bilanzkammern ausdehnen und somit ein geringfügig größeres Volumen aufnehmen können. Insbesondere bei Änderungen der Standardeinstellungen, wie zum Beispiel des Volumenstroms der Dialysierflüssigkeit, auch als DF-Fluss bezeichnet, des blutseitigen Drucks, etc. oder bei dynamischen Druckänderungen, zum Beispiel infolge einer Alterung eines DF-Filters oder ähnlichen Einheit, kann es zu Druckunterschieden und damit zu Fehlbilanzen kommen. Grund ist, dass sich entsprechend dem jeweiligen Volumenstrom die Druckbedingungen im System ändern und es entsprechend zu Volumenänderungen kommen kann, die mittels der Bilanzierungseinrichtung nicht erfasst werden können.

Ein anderer wesentlicher Nachteil ist, dass es aufgrund von Luftblasen im System zu Abweichungen bei Bilanzierungen mit volumetrischen Verfahren kommen kann. Schließlich ist es von Nachteil, dass bei bisher bekannten volumetrischen Verfahren der Flüssigkeitsentzug (Ultrafiltration) durch eine separate Pumpe erzeugt wird, was zu komplexen Blutbehandlungsvorrichtungen führt.

Die vorstehend beschriebene mangelnde Korrelation von Volumen und Gewicht wird bei bekannten gravimetrischen Verfahren vermieden. Bei diesen werden zwei Beutel (in der Regel mit einem Fassungsvermögen von mehr als 10 Litern) an einer Hängewaage befestigt. In dem einen Beutel befindet sich frische Dialysierflüssigkeit, während der andere Beutel leer ist. Während der Dialysetherapie wird die Flüssigkeit von dem einen Beutel in den anderen Beutel gepumpt. Dadurch nehmen das Gewicht des einen Beutels ab und das Gewicht des anderen Beutels zu. In der Summe bleibt das Gewicht der beiden Beutel konstant bzw. steigt mit einer Wasserentzugsrate (Ultrafiltration). Es ist ein wesentlicher Nachteil, dass bekannte gravimetrische Verfahren (Wiegeverfahren) ausschließlich einen Beutelbetrieb unterstützen. Derartige Verfahren können daher nur im Rahmen von Akutdialyseverfahren eingesetzt werden.

Bisher wurde zur Kompensation von Bilanzierungsfehlern ein UF-Kompensationsfaktor in einer Steuerungssoftware hinterlegt. Dieser Kompensationsfaktor ist jedoch unabhängig von allen vorstehend genannten variablen Parametern wie Druck, Fluss und Temperatur und stellt somit nur einen "mittleren Kompensationsfaktor" dar. Dieser ist je nach Einstellung der Maschine (Abweichungen von Standard-Dialyseeinstellungen und Standard-Umgebungsparametern) besser oder schlechter geeignet und ermöglicht eine exakte Kompensation von UF-Fehlmengen in der Regel nicht.

Zusammenfassend kann man sagen, dass es im Falle von Druck- und/oder Temperaturunterschieden in der Dialysierflüssigkeit, insbesondere vor und hinter der Bilanzkammer, zu Fehlbilanzen kommen kann. Druckunterschiede können aus verschiedenen Gründen resultieren, wie zum Beispiel Änderungen des DF-Flusses, Alterung von Komponenten (Abnutzung von DF-Filtern, Verblockung bzw. Aussetzung von Desinfektionsmitteln), Unterschieden hinsichtlich der Temperatur der Dialysierflüssigkeit relativ zur Umgebungstemperatur der Dialysemaschine, Änderungen des blutseitigen Druckes, etc. Solche Fehlbilanzierungen hängen demnach stark von an der Maschine eingestellten oder vorliegenden Parametern sowie von externen Einflussfaktoren ab.

Ausgehend von dem vorstehend beschriebenen Stand der Technik liegt der vorliegenden Erfindung die Aufgabe zugrunde, die zuvor angeführten Nachteile zu beseitigen, insbesondere eine Vorrichtung zur extrakorporalen Blutbehandlung derart auszubilden, dass eine weitgehende Unempfindlichkeit gegenüber Druck- und/oder Temperatur- und/oder Flussänderungen der Behandlungsflüssigkeit gegeben ist. Außerdem soll eine Möglichkeit geschaffen werden, eine Ultrafiltration einfach in die Vorrichtung zu integrieren. Eine Integration einer Entgasungseinrichtung sowie einer Luftabscheidevorrichtung wäre wünschenswert. Schließlich soll die Erfindung eine Kostenreduktion sowie eine Vereinfachung des Behandlungsflüssigkeitskreislaufes ermöglichen.

Nach der Erfindung wird diese Aufgabe gelöst durch eine Vorrichtung zur extrakorporalen Blutbehandlung, insbesondere eine Dialysemaschine, mit einer Internfluidik (Fluidleitungssystem innerhalb des Gehäuses einer Dialysemaschine), an die eine Blutbehandlungseinheit, insbesondere ein Dialysator, anschließbar ist, wobei die Internfluidik zumindest eine frischstromseitige Bilanzierungskammer zur Erfassung / Bilanzierung von zur Blutbehandlungseinheit fließender Frischbehandlungsflüssigkeit und zumindest eine brauchstromseitige Bilanzierungskammer zur Erfassung / Bilanzierung von von der Blutbehandlungseinheit abströmender Brauchbehandlungsflüssigkeit aufweist, wobei die Vorrichtung eine Messeinrichtung zur gravimetrischen Erfassung, insbesondere zur kontinuierlichen gravimetrischen Erfassung, von Behandlungsflüssigkeit in der frischstromseitigen und/oder eine Messeinrichtung zur gravimetrischen Erfassung, insbesondere zur kontinuierlichen gravimetrischen Erfassung, von Behandlungsflüssigkeit in der brauchstromseitigen Bilanzierungskammer aufweist.

Eine Verifizierung mit der Erfindung erfolgt dadurch, dass vor und nach einer Blutbehandlung beim Patienten das Gewicht überprüft und eine Gewichtsabweichung festgestellt wird. Durch die Erfindung erfolgt die Erfassung / Bilanzierung direkt gravimetrisch, also unmittelbar durch Erfassung des Gewichts der Behandlungsflüssigkeit. Dies hat gegenüber herkömmlichen Vorrichtungen und Bilanzierungen, die volumetrisch arbeiten bzw. erfolgen und bei denen es, wie vorstehend beschrieben wurde, zu Abweichungen kommen kann, da das Gewicht des zu bilanzierenden Volumens abhängig von der spezifischen Masse und der Temperatur des Mediums ist, den großen Vorteil, dass ein Flüssigkeitsentzug direkt mit dem Gewicht des Patienten verknüpft ist und nicht erst fehlerbehaftet umgerechnet werden muss.

Eine erfindungsgemäße Vorrichtung ist insbesondere dazu geeignet, einen kontinuierlichen Dialysierflüssigkeitsfluss gravimetrisch zu bilanzieren. Dabei liegt es außerdem im Rahmen der Erfindung, dass mit derselben Vorrichtung ohne zusätzliche Komponenten auch eine Ultrafiltration und eine Ultrafiltrationsmenge gravimetrisch erfasst / bilanziert und berechnet werden kann.

Vorteilhafte Ausführungsformen der Erfindung sind in den Unteransprüchen beansprucht und werden nachfolgend näher erläutert.

Eine Ausführungsform der Vorrichtung ist dadurch gekennzeichnet, dass die Messeinrichtung eine Waage und/oder einen Kraftsensor umfasst. Diese bzw. dieser ist zur insbesondere direkten Bestimmung der Masse / des Gewichts von in der Bilanzierungskammer befindlicher Behandlungsflüssigkeit geeignet und bestimmt. Bei einer weiteren erfindungsgemäßen Ausführungsform kann sich mindestens eine Wiegevorrichtung zum Beispiel in Form einer Waage oder in Form eines Kraftsensors als Messeinrichtung unter und/oder über der Bilanzierungskammer befinden. Die Messeinrichtung dient dazu, das Gewicht der in der jeweiligen Kammer enthaltenen Behandlungsflüssigkeit zu bestimmen. Nach der Erfindung kann die Messeinrichtung auch derart beschaffen und angeordnet sein, dass mit ihr das Gewicht der Kammer und der in ihr enthaltenen Behandlungsflüssigkeit zu bestimmen ist. Es ist besonders vorteilhaft, wenn jeder Bilanzierungskammer eine solche Messeinrichtung zugeordnet ist, sich also mindestens eine Wiegevorrichtung / Kraftsensor unter jeder Kammer befindet. Auf diese Weise kann individuell und unmittelbar die Masse / das Gewicht der in jeder Bilanzierungskammer befindlichen Behandlungsflüssigkeit erfasst und zur Bilanzierung genutzt werden.

Nach einer weiteren Ausführungsform können frischstromseitig und/oder brauchstromseitig der Behandlungseinheit jeweils zwei Bilanzierungskammern angeordnet sein, insbesondere eine erste frischstromseitige Bilanzierungskammer, eine zweite frischstromseitige Bilanzierungskammer, eine erste brauchstromseitige Bilanzierungskammer und eine zweite brauchstromseitige Bilanzierungskammer. Diese können entweder strömungstechnisch zueinander parallel oder strömungstechnisch zueinander in Serie angeordnet und miteinander verschaltet / verbunden sein. Insbesondere können nach der Erfindung die erste frischstromseitige Bilanzierungskammer und die zweite frischstromseitige Bilanzierungskammer zueinander parallel oder seriell angeordnet / verbunden / verschaltet sein. Außerdem können nach der Erfindung die erste brauchstromseitige Bilanzierungskammer und die zweite brauchstromseitige Bilanzierungskammer zueinander parallel oder seriell angeordnet / verbunden / verschaltet sein. Eine gravimetrische Bilanzierung erfolgt dann durch die insgesamt vier separaten Kammern. Das jeweilige Kammervolumen kann im Rahmen der Erfindung zum Beispiel zwischen 20 ml und 1500 ml betragen, vorzugsweise beträgt es 100 ml. Eine größere Kammer besitzt den Vorteil einer höheren Temperaturstabilität und benötigt weniger Schaltzyklen. Eine kleinere Kammer ist exakter bei der Bestimmung des bilanzierenden Gewichts, benötigt jedoch schnellere Schaltzyklen. Die einzelnen Bilanzierungskammern können wärmeisoliert ausgebildet sein.

In der Internfluidik kann bzw. können nach einer weiteren Ausführungsform zustromseitig und/oder abstromseitig der Bilanzierungskammer, insbesondere jeder der Bilanzierungskammern, jeweils ein Ventil, insbesondere ein schaltbares Absperrventil oder Flussregelventil, angeordnet sein. Vorzugsweise weist ein solches Ventil die zwei Schaltzustände "zu" und "auf" auf. Die Ventile sind jeweils insbesondere zum Steuern und/oder Regeln der Zu- bzw. Abströme von Behandlungsflüssigkeit zu und/oder aus der Bilanzierungskammer eingerichtet und bestimmt. Sie sind vorzugsweise über eine Steuerung der Vorrichtung automatisch ansteuerbar.

In einer weiteren erfindungsgemäßen Ausführungsform kann zumindest eine der Bilanzierungskammern, vorzugsweise jede Bilanzierungskammer, eine Lüftungsöffnung aufweisen. Diese kann außerdem insbesondere mit einer Filtereinheit, wie zum Beispiel mit einem Hydrophobfilter und/oder mit einem Überdruckventil versehen sein. Die Lüftungsöffnung bietet den Vorteil, dass eine Belüftung im Falle einer Entleerung sowie eine Entlüftung im Falle einer Befüllung der jeweiligen Bilanzierungskammer erfolgen können, so dass kein Überdruck bzw. Unterdruck entsteht, gegen den die Pumpen arbeiten müssen, und die Befüllungs- und Entleerungsvorgänge der Kammer besonders rasch ablaufen können. Außerdem kann über die Lüftungsöffnung Luft aus der Internfluidik entfernt werden, die sich im Laufe des Betriebs der Vorrichtung aus der darin befindlichen Behandlungsflüssigkeit abscheidet. Eine Entlüftung der Behandlungsflüssigkeit kann nach der Erfindung insbesondere erfolgen, indem in der Internfluidik insbesondere stromauf der ersten frischstromseitigen Bilanzierungskammer eine Engstelle im Strömungsquerschnitt ausgebildet ist, die zur Erzeugung eines lokalen Unterdrucks und Entgasung der Behandlungsflüssigkeit geeignet und bestimmt ist. Die Engstelle kann in Form einer Drossel, insbesondere in Form einer einstellbaren Drossel ausgebildet sein.

Eine Ausführungsform der Erfindung ist dadurch gekennzeichnet, dass die Internfluidik zustromseitig der Bilanzierungskammer und abstromseitig der Bilanzierungskammer, insbesondere jeder der Bilanzierungskammern, jeweils eine Pumpe zur Förderung von Behandlungsflüssigkeit aufweist. Insbesondere kann die Internfluidik sowohl zustromseitig und abstromseitig der frischstromseitigen Bilanzierungskammer als auch zustromseitig und abstromseitig der brauchstromseitigen Bilanzierungskammer jeweils eine Pumpe zur Förderung von Behandlungsflüssigkeit aufweisen, also insgesamt vier Pumpen, eine erste Frischstrompumpe stromauf der frischstromseitigen Bilanzierungskammer(n), eine zweite Frischstrompumpe stromab der frischstromseitigen Bilanzierungskammer(n), einer erste Brauchstrompumpe stromauf der brauchstromseitigen Bilanzierungskammer(n) und einer zweite Brauchstrompumpe stromab der brauchstromseitigen Bilanzierungskammer(n). Vorzugsweise ist zwischen jeder Pumpe und jeder Bilanzierungskammer jeweils ein Ventil, insbesondere jeweils ein schaltbares Absperrventil oder Flussregelventil, angeordnet.

Nach einer anderen Ausführungsform der Erfindung kann die Bilanzierungskammer bzw. können mehrere oder alle Bilanzierungskammern als Kolben-Zylinder-Einheit mit der Bilanzierungskammer als Zylinder und einem mit dieser zusammenwirkenden und darin aufgenommenen Kolben ausgebildet sein. Die Kolben sind nach einer Ausführungsform mittels jeweils eines Motors, insbesondere mittels jeweils eines Linearmotors, angetrieben und können derart in der Kammer auf und ab bewegt werden und einen Förderhub ausführen. Die Kolben sind an der Wand der Kammer geführt und gegenüber dieser abgedichtet. Auch im Falle von als Kolben-Zylinder-Einheit ausgebildeten Bilanzierungskammern beträgt deren Größe im Rahmen der Erfindung zwischen ca. 20ml und ca. 1500ml, insbesondere ca. 100ml. Bilanzkammern in Form von Kolben-Zylinder-Einheiten bieten unter anderem den Vorteil, dass die Masse der in der jeweiligen Bilanzierungskammer befindlichen Behandlungsflüssigkeit besser eingeregelt werden kann. Falls zum Beispiel zu viel Behandlungsflüssigkeit in eine der Bilanzierungskammern gefördert wurde und deren Soll-Gewicht überschritten ist, kann der Linearmotor durch Betrieb in umgekehrter Richtung einen Teil der Behandlungsflüssigkeit wieder zurückpumpen.

Nach der Erfindung kann die in der jeweiligen Bilanzierungskammer befindliche Behandlungsflüssigkeit mittels der Messeinrichtung kontinuierlich erfasst und insbesondere kontinuierlich bilanziert werden. Auf diese Weise kann mit Vorteil besonders einfach regelnd oder steuernd in das Verfahren eingegriffen werden.

Nach der Erfindung kann insbesondere in einer ersten Phase des Verfahrens frischstromseitig frische Behandlungsflüssigkeit in die erste frischstromseitige Bilanzierungskammer gefördert werden, während frische Behandlungsflüssigkeit aus der zweiten frischstromseitigen Bilanzierungskammer zur Behandlungseinheit gefördert wird. Derweil wird brauchstromseitig gebrauchte Behandlungsflüssigkeit aus der Behandlungseinheit in die erste brauchstromseitige Bilanzierungskammer gefördert, während gebrauchte Behandlungsflüssigkeit aus der zweiten brauchstromseitigen Bilanzierungskammer zu einem Ablauf der Vorrichtung gefördert wird. In einer zweiten Phase des Verfahrens wird frischstromseitig frische Behandlungsflüssigkeit in die zweite frischstromseitige Bilanzierungskammer gefördert, während frische Behandlungsflüssigkeit aus der ersten frischstromseitigen Bilanzierungskammer zur Behandlungseinheit gefördert wird. Brauchstromseitig wird derweil gebrauchte Behandlungsflüssigkeit aus der Behandlungseinheit in die zweite brauchstromseitige Bilanzierungskammer gefördert, während gebrauchte Behandlungsflüssigkeit aus der ersten brauchstromseitigen Bilanzierungskammer zu einem Ablauf der Vorrichtung gefördert wird. Bei dieser Ausführungsform sind die erste und die zweite frischstromseitige Bilanzierungskammern sowie die erste und die zweiter brauchstromseitige Bilanzierungskammern jeweils zueinander parallel angeordnet / strömungstechnisch miteinander verknüpft.

In einer alternativen Ausführungsform wird in einer ersten Phase des Verfahrens frischstromseitig frische Behandlungsflüssigkeit in die erste frischstromseitige Bilanzierungskammer gefördert, während frische Behandlungsflüssigkeit aus der zweiten frischstromseitigen Bilanzierungskammer zur Behandlungseinheit gefördert wird. Zeitgleich wird brauchstromseitig gebrauchte Behandlungsflüssigkeit aus der Behandlungseinheit in die erste brauchstromseitige Bilanzierungskammer gefördert und gebrauchte Behandlungsflüssigkeit aus der zweiten brauchstromseitigen Bilanzierungskammer zu einem Ablauf der Vorrichtung gefördert. In dieser ersten Phase sind die erste und die zweite frischstromseitige Bilanzierungskammer wie auch die erste und die zweite brauchstromseitige Bilanzierungskammer strömungstechnisch voneinander entkoppelt, zum Beispiel durch ein zwischengeschaltetes Absperrventil. In einer zweiten Phase des Verfahrens wird dann frischstromseitig frische Behandlungsflüssigkeit aus der ersten frischstromseitigen Bilanzierungskammer in die zweite frischstromseitige Bilanzierungskammer gefördert. Zeitgleich kann außerdem frische Behandlungsflüssigkeit aus der zweiten frischstromseitigen Bilanzierungskammer zur Behandlungseinheit gefördert werden. Brauchstromseitig wird gebrauchte Behandlungsflüssigkeit aus der Behandlungseinheit in die erste brauchstromseitige Bilanzierungskammer gefördert. Zeitgleich kann außerdem gebrauchte Behandlungsflüssigkeit aus der zweiten brauchstromseitigen Bilanzierungskammer zu einem Ablauf der Vorrichtung gefördert werden. In dieser zweiten Phase des Verfahrens sind die erste und die zweite frischstromseitige Bilanzierungskammer wie auch die erste und die zweite brauchstromseitige Bilanzierungskammer strömungstechnisch miteinander gekoppelt, zum Beispiel durch Durchschalten des zwischengeschalteten Absperrventils. Bei dieser Ausführungsform sind die erste und die zweite frischstromseitige Bilanzierungskammer sowie die erste und die zweiter brauchstromseitige Bilanzierungskammer jeweils zueinander seriell angeordnet / strömungstechnisch miteinander verknüpft.

Zusammenfassend kann man sagen, dass durch die Erfindung insbesondere die folgenden Vorteile und Verbesserungen erzielt werden können:
- Die Vorrichtung nach der Erfindung eignet sich insbesondere für eine gravimetrisch kontinuierliche Bilanzierung und für eine Bilanzierung eines kontinuierlichen Flusses.
- Die Vorrichtung nach der Erfindung eignet sich insbesondere für eine Bilanzierung einer Ultrafiltrationsmenge, ohne dass dazu eine eigenständige separate Ultrafiltrationspumpe erforderlich ist.
- Die Bilanzierung ist robust gegen Druck- und/oder Durchfluss und/oder Temperaturänderungen der Behandlungsflüssigkeit.
- Für eine korrekte Bilanzierung ist nicht mehr erforderlich, dass der Druck auf den jeweiligen Eingangs- und Ausgangsseiten der Bilanzierungskammern konstant und/oder identisch ist oder gehalten werden muss.
- Die Vorrichtung nach der Erfindung eignet sich insbesondere sowohl zur Implementierung einer Entgasung (Wassereingang) als auch zur Implementierung eines Luftabscheiders (Dialysatorausgang), so dass keine eigenständige, separate Entgasungspumpe und/oder Luftabscheider erforderlich ist.
- Die Vorrichtung nach der Erfindung ist insbesondere gegenüber Bilanzierungsfehlern durch Luftblasen robust.
- Die Vorrichtung nach der Erfindung eignet sich insbesondere für einen Selbsttest oder Abgleich. Zwei frischstromseitige Bilanzierungskammern können zum Beispiel getestet oder abgeglichen werden, indem darin über diesen zugeordnete Pumpen jeweils unterschiedliche Füllstände / Füllmengen eingestellt, die Pumpen angehalten und alle frischstromseigen Kammerventile geöffnet werden. Zwei brauchstromseitige Bilanzierungskammern können zum Beispiel getestet oder abgeglichen werden, indem darin über diesen zugeordnete Pumpen jeweils unterschiedliche Füllstände / Füllmengen eingestellt, die Pumpen angehalten und alle brauchstromseigen Kammerventile geöffnet werden. Über die geöffneten Kammerventile stellen sich in den jeweiligen Bilanzierungskammern gleiche Pegel und somit gleiche Füllmengen (Gewicht) ein. Eine Möglichkeit für einen Selbsttest oder Abgleich einer frischstromseitigen Bilanzierungskammer mit einer brauchstromseitigen Bilanzierungskammer kann im Rahmen der Erfindung dadurch erfolgen, dass die frischstromseitige Bilanzierungskammer mit einer bestimmten Menge an Behandlungsflüssigkeit gefüllt und die brauchstromseitige Bilanzierungskammer bis auf eine bestimmte Restmenge an Behandlungsflüssigkeit geleert wird. Danach wird Behandlungsflüssigkeit aus der frischstromseitigen Bilanzierungskammer in die brauchstromseitige Bilanzierungskammer gepumpt. Nachdem in der frischstromseitigen Bilanzierungskammer nur noch eine der Restmenge in der brauchstromseitigen Bilanzierungskammer zu Beginn des Abgleichs entsprechende Restmenge an Behandlungsflüssigkeit enthalten ist, muss sich das Gewicht der in der brauchstromseitigen Bilanzierungskammer befindlichen Behandlungsflüssigkeit auf das Ausgangsgewicht der frischstromseitigen Bilanzierungskammer erhöht haben.

Die Erfindung sowie ein Beispiel zum Stand der Technik werden im Folgenden anhand beispielhafter, nicht einschränkender und in den angehängten Figuren gezeigter Ausführungsformen näher erläutert. Dabei zeigt:
Fig. 1 ein Schema eines Ausschnitts einer bekannten Vorrichtung zur extrakorporalen Blutbehandlung,
Fig. 2 ein Schema eines eine Bilanzierungseinheit bildenden Ausschnitts einer ersten Ausführungsform einer erfindungsgemäßen Vorrichtung zur extrakorporalen Blutbehandlung,
Fig. 3 ein Schema eines eine Bilanzierungseinheit bildenden Ausschnitts einer zweiten Ausführungsform einer erfindungsgemäßen Vorrichtung zur extrakorporalen Blutbehandlung,
Fig. 4 ein Schema eines eine Bilanzierungseinheit bildenden Ausschnitts einer dritten Ausführungsform einer erfindungsgemäßen Vorrichtung zur extrakorporalen Blutbehandlung,
Fig. 5 ein Schema eines eine Bilanzierungseinheit bildenden Ausschnitts einer vierten Ausführungsform einer erfindungsgemäßen Vorrichtung zur extrakorporalen Blutbehandlung,
Fig. 6 eine schematische perspektivische Darstellung einer Bilanzierungseinheit einer Ausführungsform einer erfindungsgemäßen Vorrichtung zur extrakorporalen Blutbehandlung,
Fig. 7 eine schematische perspektivische Darstellung einer Bilanzierungseinheit einer Ausführungsform einer erfindungsgemäßen Vorrichtung zur extrakorporalen Blutbehandlung, und
Fig. 8 eine schematische perspektivische Darstellung einer Bilanzierungseinheit einer Ausführungsform einer erfindungsgemäßen Vorrichtung zur extrakorporalen Blutbehandlung.

Figur 2 zeigt schematisch einen Ausschnitt einer ersten Ausführungsform einer erfindungsgemäßen Vorrichtung 1 zur extrakorporalen Blutbehandlung. Im vorliegenden Beispiel ist die Vorrichtung 1 als Dialysemaschine 1 ausgebildet und umfasst eine Internfluidik 2, an die eine Blutbehandlungseinheit 3, hier im vorliegenden Fall ein Dialysator 3, anschließbar ist. Die Internfluidik 2 umfasst eine erste frischstromseitige Bilanzierungskammer 4 und eine zweite frischstromseitige Bilanzierungskammer 5 jeweils zur Bilanzierung von zur Blutbehandlungseinheit 3 fließender Frischbehandlungsflüssigkeit. Sie umfasst außerdem eine erste brauchstromseitige Bilanzierungskammer 6 und eine zweite brauchstromseitige Bilanzierungskammer 7 jeweils zur Bilanzierung von von der Blutbehandlungseinheit 3 abströmender Brauchbehandlungsflüssigkeit. Die einzelnen Bilanzierungskammern 4, 5, 6, 7 können wärmeisoliert sein.

Frischstromseitig weist die Internfluidik 2 eine mit einem nicht dargestellten Reservoir für frische Behandlungsflüssigkeit verbundene Zuflussleitung 8 auf. In dieser ist eine erste Frischstrompumpe 9 in der Internfluidik 2 angeordnet. Stromab der Pumpe 9 teilt sich die Leitung 8 in einen ersten Frischstromleitungsstrang 10 und einen zweiten Frischstromleitungsstrang 11 auf, die zueinander strömungstechnisch parallel ausgebildet sind.

Im ersten Frischstromleitungsstrang 10 ist stromauf der ersten frischstromseitigen Bilanzierungskammer 4 ein Ventil 12 und stromab der ersten frischstromseitigen Bilanzierungskammer 4 ein Ventil 13 angeordnet. Im zweiten Frischstromleitungsstrang 11 ist stromauf der zweiten frischstromseitigen Bilanzierungskammer 5 ein Ventil 14 und stromab der zweiten frischstromseitigen Bilanzierungskammer 5 ein Ventil 15 angeordnet. Stromab der beiden Ventile 13, 15 vereinen sich der erste Frischstromleitungsstrang 10 und der zweite Frischstromleitungsstrang 11 wieder zu einer Frischstromleitung 16, in deren weiteren Verlauf eine zweite Frischstrompumpe 17 angeordnet ist und die schließlich mit dem Dialysator 3 als Behandlungseinheit 3 verbunden ist.

Brauchstromseitig weist die Internfluidik 2 einen mit dem Dialysator 3 strömungstechnisch verbundene Brauchstromleitung 18 auf. In dieser ist eine erste Brauchstrompumpe 19 angeordnet. Stromab der ersten Brauchstrompumpe 19 teilt sich die Brauchstromleitung 18 in einen ersten Brauchstromleitungsstrang 20 und einen zweiten Brauchstromleitungsstrang 21 auf, die zueinander strömungstechnisch parallel ausgebildet sind.

Im ersten Brauchstromleitungsstrang 20 ist stromauf der ersten brauchstromseitigen Bilanzierungskammer 6 ein Ventil 22 und stromab der ersten brauchstromseitigen Bilanzierungskammer 6 ein Ventil 23 angeordnet. Im zweiten Brauchstromleitungsstrang 21 ist stromauf der zweiten brauchstromseitigen Bilanzierungskammer 7 ein Ventil 24 und stromab der zweiten brauchstromseitigen Bilanzierungskammer 7 ein Ventil 25 angeordnet. Stromab der beiden Ventile 23, 25 vereinen sich der erste Brauchstromleitungsstrang 20 und der zweite Brauchstromleitungsstrang 21 wieder zu einer Brauchstromleitung 26, in deren weiteren Verlauf eine zweite Brauchstrompumpe 27 angeordnet ist und die schließlich über einen Abfluss 28 mit einem nicht dargestellten Reservoir für gebrauchte Behandlungsflüssigkeit verbunden ist.

Dargestellt in Figur 2 ist außerdem ein Teil einer extrakorporalen Blutleitung 29 mit angedeuteter Blutflussrichtung 30.

Bei der Vorrichtung der Figur 2 werden insgesamt vier Kammern 4, 5, 6, und 7 eingesetzt, die jeweils wärmeisoliert sein können. Die Umschaltrate der Kammern 4, 5, 6, 7 erfolgt abhängig von den jeweiligen Volumina der Kammern. Wie in Figur 2 angedeutet ist, sind der erste Frischstromleitungsstrang 10, der zweite Frischstromleitungsstrang 11, der erste Brauchstromleitungsstrang 20 und der zweite Brauchstromleitungsstrang 21 möglichst in Bodennähe der jeweiligen Kammer 4, 5, 6, 7 mit dieser verbunden, damit möglichst keine Toträume innerhalb der jeweiligen Kammer 4, 5, 6, 7 bleiben, in denen sich Behandlungsflüssigkeit anstauen kann.

Jede der vier Kammern 4, 5, 6, 7 verfügt über eine Lüftungsöffnung 31, vorliegend angeordnet an der Oberseite der jeweiligen Kammer 4, 5, 6, 7. Die Lüftungsöffnung 31 dient einem Druckausgleich bei Befüllen sowie Entleeren der jeweiligen Kammer 4, 5, 6, 7. Die Lüftungsöffnung 31 kann mit einem in der Figur nicht gezeigten Hydrophobfilter versehen sein.

Unterhalb jeder einzelnen Kammer 4, 5, 6, 7 befindet sich eine Messeinrichtung 32 hier in Form einer Waage oder Wiegeeinrichtung 32. Alternativ kann die Messeinrichtung 32 oberhalb der jeweiligen Kammer 4, 5, 6, 7 angeordnet sein. Die Messeinrichtung 32 ist dazu geeignet und bestimmt, kontinuierlich das Gewicht der jeweiligen mit mehr oder weniger Behandlungsflüssigkeit gefüllten Kammer 4, 5, 6, 7 zu ermitteln. Die bestimmten Gewichte können genutzt werden, um den Massenstrom der Pumpen 9, 17, 19, 27 zu bestimmen. Des Weiteren dient es der Bestimmung des Füllgrades der Kammern 4, 5, 6, 7.

Zum Bewirken eines kontinuierlichen Flusses von Behandlungsflüssigkeit unterteilt sich deren Förderung in zwei Phasen. Zu Beginn einer ersten Phase sind die Kammern 4, 5, 6, 7 bei einem jeweiligen Füllvolumen von 100ml mit den folgenden Massen an Behandlungsflüssigkeit gefüllt:
Kammer 4: 20g, Kammer 5: 80g, Kammer 6: 20g, Kammer 7: 80g

In der ersten Phase wird die Vorrichtung 1 wie folgt betrieben:
Die Pumpe 9 füllt durch das Ventil 12 die Kammer 4. Das Ventil 12 ist offen, das Ventil 13 geschlossen.

Die Pumpe 17 leert durch das Ventil 15 die Kammer 5. Das Ventil 14 ist geschlossen, das Ventil 15 offen.

Die Pumpe 19 füllt durch das Ventil 22 die Kammer 6. Das Ventil 23 ist geschlossen, das Ventil 22 offen.

Die Pumpe 27 leert durch das Ventil 25 die Kammer 7. Das Ventil 25 ist offen, das Ventil 24 geschlossen.

Der Betrieb in der ersten Phase erfolgt so lange, bis die Kammern 4, 5, 6, 7 die Zustände voll bzw. leer erreicht haben.

Zu Beginn einer zweiten Phase sind die Kammern 4, 5, 6, 7 bei einem jeweiligen Füllvolumen von 100ml mit den folgenden Massen an Behandlungsflüssigkeit gefüllt: Kammer 4 80g, Kammer 5 20g, Kammer 6 80g, Kammer 7 20g

In der zweiten Phase wird die Vorrichtung 1 wie folgt betrieben:
Die Pumpe 9 füllt durch das Ventil 14 die Kammer 5. Das Ventil 14 ist offen, das Ventil 15 ist geschlossen.

Die Pumpe 17 leert durch das Ventil 13 die Kammer 4. Das Ventil 12 ist geschlossen, das Ventil 13 offen,
Die Pumpe 19 füllt durch das Ventil 24 die Kammer 7. Das Ventil 25 ist geschlossen, das Ventil 24 offen.

Die Pumpe 27 leert durch das Ventil 23 die Kammer 6. Das Ventil 23 ist offen, das Ventil 22 geschlossen.

Auch der Betrieb in der zweiten Phase erfolgt so lange, bis die Kammern 4, 5, 6, 7 die Zustände voll bzw. leer erreicht haben.

Sobald der jeweilige Umschaltpunkt (leer oder voll) einer Kammer 4, 5, 6, 7 erreicht wurde, schließen die beiden zur Kammer 4, 5, 6, 7 gehörenden Ventile. Sobald alle Ventile aller Kammern 4, 5, 6, 7 geschlossen sind, beginnt die nächste Phase, also nach der ersten Phase die zweite Phase, dann wieder die erste Phase und so weiter.

Die Vorrichtung der Figur 2 kann im Rahmen der Erfindung auch in einem Ultrafiltrationsmodus betrieben werden, also derart, dass einem Patienten in einer gewünschten Weise und in einem gewünschten Maß Flüssigkeit entzogen wird. Um eine Ultrafiltration zu generieren, werden die oberen Umschaltpunkte der Kammern 4, 5, 6, 7 unterschiedlich definiert und werden die Frischstrompumpe 17 und die Brauchstrompumpe 19 mit unterschiedlichen Förderraten betrieben. Im vorliegenden Beispiel läuft die Frischstrompumpe 17 mit einer Förderrate von 500g/min, welche Förderrate mittels der Messeinrichtung bestimmt wird. Die Brauchstrompumpe 19 wird mit einer unterschiedlichen Förderrate von 510g/min betrieben, so dass sich insgesamt eine Ultrafiltration von 600g/h ergibt. Bei solchen Förderraten ergeben sich für die Kammern 4 und 5 die Umschaltpunkte 20g und 80g (was einem Hub von 60g bei 8,33 Hüben pro Minute entspricht). Für die Kammern 6 und 7 ergeben sich die Umschaltpunkte 20g und 81,2g (was einem Hub von 61.2g bei 8,33 Hüben pro Minute entspricht).

Die Frischstrompumpe 9 und die Brauchstrompumpe 27 müssen jeweils derart betrieben werden, dass die jeweils mit ihnen strömungstechnisch verbundenen Kammern mindestens genauso schnell gefüllt bzw. geleert werden, wie die Frischstrompumpe 7 und die Brauchstrompumpe 19 benötigen, um die jeweils andere mit ihnen strömungstechnisch verbundene Kammer zu leeren bzw. zu füllen.

Außerdem ist die Vorrichtung des Ausführungsbeispiels der Figur 2 eingerichtet für eine Entgasung der Behandlungsflüssigkeit. In der Zuflussleitung 8 ist eine Engstelle 33 oder Drossel 33 angeordnet, die in der durch sie hindurchströmenden Behandlungsflüssigkeit einen dynamischen Unterdruck erzeugt. Infolge des dort vorliegenden Unterdrucks scheidet sich in der Behandlungsflüssigkeit gelöste Luft in Form von Luftblasen ab, die dann durch die Lüftungsöffnungen 31 in der Bilanzierungskammer 4 bzw. 5 entweichen können. Luft in Form von Luftblasen, die z.B. durch Leckagen an den Dialysatorkupplungen in das System eindringen, kann bei der erfindungsgemäßen Vorrichtung 1 einfach über die Bilanzierungskammern 6 und 7 aus der Internfluidik ausgeschieden werden. Ein separater Luftabscheider für diesen Zweck ist bei der Vorrichtung 1 der Erfindung in vorteilhafter Weise nicht erforderlich.

Ein Abgleich der Bilanzierungskammern 4 und 5 bzw. 6 und 7 ist bei der erfindungsgemäßen Vorrichtung 1 besonders einfach möglich. Über die Pumpen 9, 17, 19, 27 werden in den Bilanzierungskammern 4, 5, 6, 7 jeweils unterschiedliche Füllstände / Füllmengen eingestellt, beispielsweise 20g, 50g und 80g. Anschließend werden die Pumpen 9, 17, 19, 27 angehalten und alle Kammerventile 12, 13,14, 15 und 22, 23, 24, 25 geöffnet. Über die geöffneten Kammerventile 12, 13, 14, 15 und 22, 23, 24, 25 stellen sich in den Bilanzierungskammern 4 und 5 bzw. 6 und 7 gleiche Pegel und somit gleiche Füllmengen (Gewicht) ein.

Außerdem kann ein Abgleich der Bilanzierungskammern 4 mit 6 (oder 4 mit 7, 5 mit 6, 5 mit 7) in einfacher Weise wie folgt erfolgen: Über die Pumpen 9, 17, 19, 27 werden die Bilanzierungskammer 4 gefüllt (z. B. mit 80g Behandlungsflüssigkeit) und die Bilanzierungskammer 6 geleert (z. B. auf eine Menge von 20g Behandlungsflüssigkeit). Danach wird die Behandlungsflüssigkeit aus der Bilanzierungskammer 4 in die Bilanzierungskammer 6 gepumpt. Dies kann in einer ersten Varianten mit nur einer Pumpe erfolgen, indem eine der Pumpen 17 und 19 über einen in der Figur 2 nicht gezeigten Bypass überbrückt wird, sodass die verbleibende Pumpe 17 bzw. 19 direkt einen Fluss zwischen den Bilanzierungskammern 4 und 6 herstellt. Alternativ kann dies in einer zweiten Variante mit zwei Pumpen erfolgen, indem die Pumpen 17 und 19 derart betrieben werden, dass beide die gleiche Förderrate aufweisen. Dazu kann z. B. ein in der Figur 2 nicht dargestellter Drucksensor zwischen den beiden Pumpen 17 und 19 eingesetzt sein. Werden die Pumpen 17 und 19 dann mit einer solchen Drehzahl betrieben, dass sich dieser Druck nicht ändert, ist sichergestellt, dass beide Pumpen 17 und 19 die gleiche Menge an Behandlungsflüssigkeit fördern. Nachdem in der Bilanzierungskammer 4 das untere Niveau von 20g Behandlungsflüssigkeit erreicht ist, muss sich das Gewicht der in der Bilanzierungskammer 6 befindlichen Behandlungsflüssigkeit auf das Ausgangsgewicht der Bilanzierungskammer 4, also hier 80g, erhöht haben.

Schließlich ermöglicht die Vorrichtung 1 nach der Erfindung einen gleichzeitigen Abgleich aller Bilanzierungskammern 4, 5, 6, 7. Durch eine Integration eines in Figur 2 nicht dargestellten Kurzschlussventils zwischen z. B. den Ventilen 14 und 15 kann der vorstehend beschriebene Abgleichvorgang für alle Bilanzierungskammern 4, 5, 6,7 gleichzeitig durchgeführt werden.

In einer erfindungsgemäßen Ausführungsform kann eine Absicherung der Messergebnisse mittels einer zweikanaligen Messung sichergestellt werden. Dies kann zum Beispiel erfolgen, indem eine zusätzliche Differenzmessung zwischen den jeweiligen Bilanzierungskammern vor und nach dem Dialysator 3 durch ein Wiegen der Kammern (z.B. Kammer 4 mit Kammer 6 und Kammer 5 mit Kammer 7) durchgeführt wird, insbesondere mit Hilfe einer in den Figuren nicht gezeigten Wippvorrichtung, auf der die entsprechenden Kammern aufgebracht sind. Alternativ kann das Gesamtgewicht zweier Kammern, zum Beispiel der Bilanzierungskammern 4 und 6 sowie der Bilanzierungskammern 5 und 7 auf einer ebenfalls in den Figuren nicht dargestellten Waage festgestellt und mittels Differenzbildung mit den Ergebnissen der Einzelwaagen eine Bilanzierung durchgeführt werden.

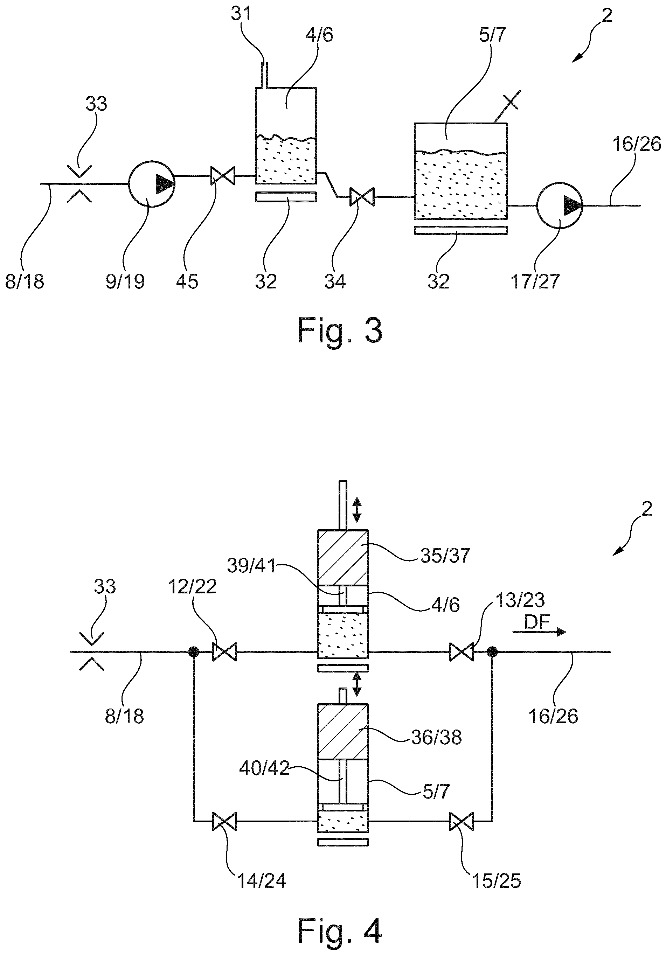

Anhand der Figuren 2 und 3 kann eine zweite Ausführungsvariante der Erfindung verdeutlicht werden. Die zweite Ausführungsform unterscheidet sich von der ersten in Figur 2 gezeigten ersten Ausführungsform dadurch, dass die in Figur 2 innerhalb der gestrichelten Umrandungen befindlichen Bestandteile jeweils durch die in Figur 3 gezeigten Bestandteile ersetzt sind. Insbesondere die Bilanzierungskammern 4, 5, 6, 7 sind in beiden Ausführungsformen identisch ausgebildet. Bei der folgenden Beschreibung der zweiten Ausführungsform wird lediglich die Frischstromseite der Vorrichtung 1 beschrieben und darauf hingewiesen, dass die Brauchstromseite der Vorrichtung entsprechen ausgebildet und gegenüber der ersten Ausführungsform der Figur 2 modifiziert ist.

Bei dieser Ausführungsform der Vorrichtung 1 sind die Bilanzierungskammern 4 und 5 bzw. 6 und 7 nicht zueinander parallel, sondern in Reihe angeordnet. Die Bilanzierungskammern 5 und 6 weisen in etwa das doppelte oder mehr als das doppelte Volumen wie die mit ihnen jeweils in Reihe angeordneten Bilanzierungskammern 4 und 7 auf. Frischstromseitig fördert die Frischstrompumpe 17 kontinuierlich Behandlungsflüssigkeit aus der zweiten Bilanzierungskammer 5 durch den Dialysator 3. Mit Hilfe der Messeinrichtung 32 oder alternativ eines Flusssensors kann der durch diese Pumpe 17 hervorgerufene (Masse-)-Fluss bestimmt werden. Die erste frischstromseitige Bilanzierungskammer 4 dient der eigentlichen Bilanzierung. Das Gewicht der in ihr befindlichen Behandlungsflüssigkeit wird kontinuierlich mittels der unter der Kammer 4 befindlichen Messeinrichtung 32 gemessen. Die erste frischstromseitige Bilanzierungskammer 4 besitzt, ähnlich wie in der ersten Ausführungsform der Figur 2, zwei Umschaltpunkte leer (z B. 20g) und voll (z.B. 80g). Ist die Bilanzierungskammer 4 leer (enthält also nur noch 20g Flüssigkeit), öffnet das Ventil 45, während das Ventil 34 schließt, so dass die erste Frischstrompumpe 9 die erste frischstromseitige Bilanzierungskammer 4 füllt. Da die Lüftungsöffnung 31 der Bilanzierungskammer 4 dann geschlossen ist, wird in der Kammer 4 befindliche Luft dabei komprimiert und der Druck in der Kammer 4 steigt an. Sobald die erste frischstromseitige Bilanzierungskammer 4 voll ist (also 80g Behandlungsflüssigkeit enthält), schließt das Ventil 45 und öffnet das Ventil 34. Durch den in der ersten frischstromseitigen Bilanzierungskammer 4 aufgebauten Druck (oder aufgrund eines gegebenenfalls zwischen den Kammern 4 und 5 vorliegenden Höhenunterschieds) strömt Behandlungsflüssigkeit aus der ersten frischstromseitigen Bilanzierungskammer 4 in die zweite frischstromseitige Bilanzierungskammer 5. Sobald die erste Kammer 4 leer ist (also nur noch 20g Behandlungsflüssigkeit enthält), schließt das Ventil 34 und öffnet das Ventil 45 und der Zyklus beginnt von vorn. Ist sichergestellt, dass Behandlungsflüssigkeit in einem ausreichenden Maße oder häufig genug aus der ersten frischstromseitigen Bilanzierungskammer 4 in die zweite frischstromseitige Bilanzierungskammer 5 strömt, wird diese niemals vollständig entleert. Somit kann die Pumpe 17 einen kontinuierlichen Fluss durch den Dialysator 3 bereitstellen. Es sei darauf hingewiesen, dass für eine Bilanzierung nur die Messeinrichtung 32 unter der ersten Bilanzierungskammer (4, 6) ausreichend ist.

Auch die Vorrichtung 1 nach der zweiten Ausführungsform ist für eine Entgasung geeignet und eingerichtet, indem durch eine Verengung 33 in der Internfluidik 2, hier vor der Frischstrompumpe 9, ein Unterdruck in der Behandlungsflüssigkeit erzeugt wird. Sich dadurch bildende Luftblasen werden sich in der ersten frischstromseitigen Bilanzierungskammer 4 sammeln, wodurch der Druck darin ansteigt. Dieses erhöht die Ausflussgeschwindigkeit der Behandlungsflüssigkeit aus der Bilanzierungskammer 4 in die Bilanzierungskammer 5. Zur Vermeidung von zu hohen Drücken in der Kammer 4 ist diese mit einem in der Lüftungsöffnung 31 angeordneten und in der Figur 3 nicht gezeigtem Überdruckventil versehen.

Gegenüber der ersten Ausführungsform (Figur 2) besitzt die zweite Ausführungsform der Erfindung den Vorteil, dass weniger Ventile erforderlich sind. Allerdings ist gegenüber der ersten Ausführungsform und der im Nachfolgenden noch beschriebenen dritten Ausführungsform das Kammervolumen der Bilanzierungskammern 5 und 7 größer.

Anhand der Figuren 2 und 4 kann eine dritte Ausführungsvariante der Erfindung verdeutlicht werden. Die dritte Ausführungsform unterscheidet sich von der ersten in Figur 2 gezeigten ersten Ausführungsform dadurch, dass die in Figur 2 innerhalb der gestrichelten Umrandungen befindlichen Bestandteile jeweils durch die in Figur 4 gezeigten Bestandteile ersetzt sind. Bei der folgenden Beschreibung der dritten Ausführungsform wird lediglich die Frischstromseite der Vorrichtung 1 beschrieben und darauf hingewiesen, dass die Brauchstromseite der Vorrichtung entsprechen ausgebildet und gegenüber der ersten Ausführungsform der Figur 2 modifiziert ist.

Im Vergleich zur ersten Ausführungsform sind deren Pumpen 9, 17, 19 und 27 durch entsprechend angeordnete Linearmotoren 35, 36, 37, 38 ersetzt. Die Linearmotoren 35, 36, 37, 38 wirken mit in den entsprechenden Bilanzierungskammern 4, 5, 6, 7 beweglich angeordneten Kolben 39, 40, 41, 42 zusammen. Die Kolben 39, 40, 41, 42 sind in der entsprechenden jeweiligen Kammer 4, 5, 6, 7 geführt und gegenüber der Kammerwand abgedichtet, so dass die Kammern 4, 5, 6, 7 jeweils einen zum Kolben 39, 40, 41, 42 gehörenden Zylinder ausbilden. Durch eine mittels der Linearmotoren 35, 36, 37, 38 bewirkte Bewegung der Kolben 39, 40, 41, 42 in den Kammern 4, 5, 6, 7 werden diese gefüllt und entleert. Die Kolben können im Rahmen der Erfindung sowohl hängend als auch stehend angeordnet sein. Die einzelnen Bilanzierungskammern 4, 5, 6, 7 können wärmeisoliert sein. Ihre Größe beträgt im Rahmen der Erfindung zwischen ca. 20ml bis ca. 1500ml. In Abhängigkeit von der Größe der Kammern 4, 5, 6, 7 ändert sich der Schaltzyklus der Kolben 39, 40, 41, 42.

Die in Figur 4 gezeigte dritte Ausführungsform besitzt gegenüber der in Figur 2 gezeigten ersten Ausführungsform den Vorteil, dass die Masse der in der jeweiligen Kammer 4, 5, 6, 7 befindlichen Behandlungsflüssigkeit besser eingeregelt werden kann. Falls das Soll-Gewicht überschritten wird, also sich in der jeweiligen Kammer 4, 5, 6, 7 zu viel Behandlungsflüssigkeit befindet, kann der Linearmotor 35, 36, 37, 38 einen Teil der Behandlungsflüssigkeit wieder zurückpumpen. Nachteilig gegenüber der ersten Ausführungsform ist, dass keine Entlüftung der Kammern 4, 5, 6, 7 möglich ist, jeweils jeder Linearmotor 35, 36, 37, 38 sowie der dazugehörige Kolben 39, 40, 41, 42 bei der Bilanzierung mittels der Messeinrichtung 32 zusätzlich gewogen werden und der Schweredruck (hydrostatischer Druck) nicht entkoppelt ist und daher wird durch die Messeinrichtung 2 mitgemessen wird.

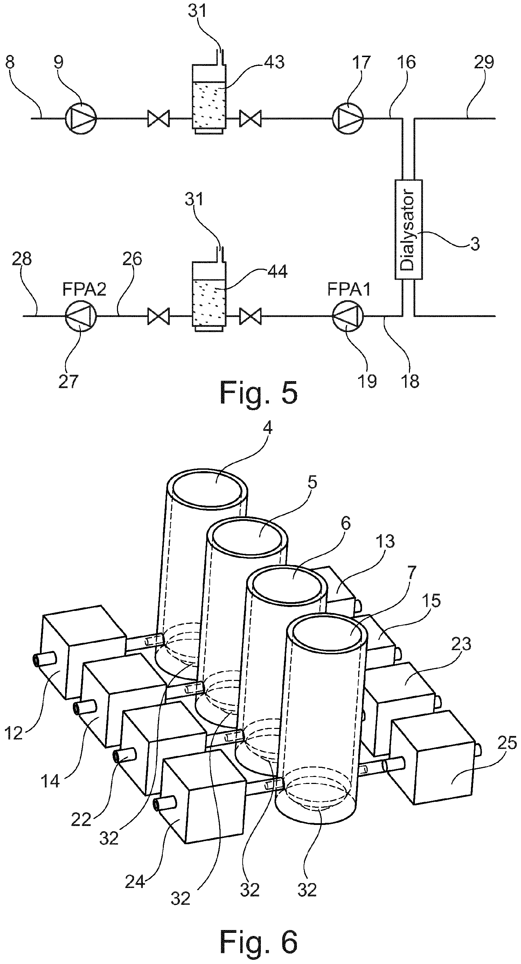

Eine vierte Ausführungsform der Vorrichtung 1 nach der Erfindung ist in Figur 5 dargestellt. Bei dieser ist auf der Frischstromseite (stromauf des Dialysators 3) nur eine (einzige) frischstromseitige Bilanzierungskammer 43 sowie auf Brauchstromseite (stromab des Dialysators 3) nur eine (einzige) brauchstromseitige Bilanzierungskammer 44 angeordnet. Die erste Frischstrompumpe 9 füllt die frischstromseitige Bilanzierungskammer 43 sehr schnell (z.B. mit dem 10-fachen Dialysatfluss). Nach Beenden des Füllvorgangs fördert die zweite Frischstrompumpe 17 Behandlungsflüssigkeit aus Bilanzierungskammer 43 mit einer konstanten Förderrate, bis die Kammer 43 leer ist. In analoger Weise befüllt die erste Brauchstrompumpe 19 die brauchstromseitige Bilanzierungskammer 44 mit einer konstanten Förderrate. Wenn die Bilanzierungskammer 44 voll ist, entleert die zweite Brauchstrompumpe 27 die Kammer 44 mit einer hohen Förderrate (z.B. 10-fache Förderrate). Auf diese Weise wird besonders einfach ein quasi-kontinuierlicher Dialysatfluss erzeugt. Der Zyklus wiederholt sich periodisch. Die Messung und Bilanzierung erfolgt analog zu den zuvor beschriebenen Ausführungsformen.

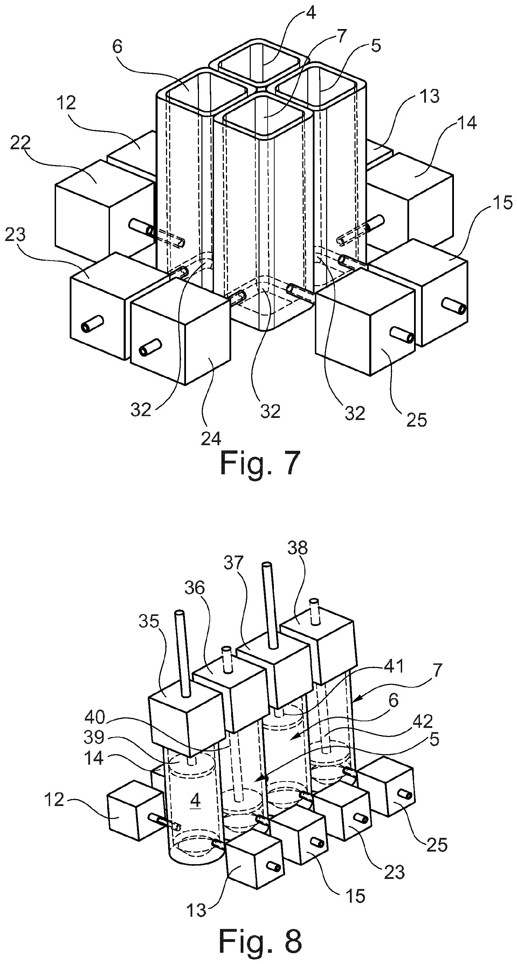

Die Figuren 6 und 7 zeigen mögliche Ausgestaltungen der ersten und zweiten Ausführungsformen der Erfindung. Es handelt sich um vier separate Kammern 4, 5, 6, 7, unter denen jeweils eine Messeinrichtung 32 angebracht ist. Die Ventile 12, 13, 14, 15 und 22, 23, 24, 25 sind bei beiden Ausführungsbeispielen separat von den Kammern 4, 5, 6, 7 angebracht, um die Kammern 4, 5, 6, 7 weitestgehend mechanisch zu entkoppeln. So übertragen sich z.B. Ventilschaltungen nicht direkt auf die Messeinrichtungen 32, hier in Form von Kraftsensoren 32.

Alle Kammern 4, 5, 6,7 sind nach oben offen dargestellt, sie sind allerdings durch einen in den Figuren 6 und 7 nicht gezeigten Deckel beispielsweise mit einem Überlaufstutzen und/oder einem Hydrophobfilter versehen.

Die Figur 8 schließlich zeigt eine mögliche Umsetzung der dritten Ausführungsform. Diese beruht auf der in Figur 6 gezeigten Umsetzung. Die Kammern 4, 5, 6, 7 wurden durch die Linearmotoren 35, 36, 37, 38 ergänzt. Diese bewegen jeweils einen der Kolben 39, 40, 41, 42 in der Kammer 4, 5, 6, 7 auf und ab, wodurch der gewünschte Fluss an Behandlungsflüssigkeit entsteht.

### Bezugszeichen

- 1: Vorrichtung zur extrakorporalen Blutbehandlung, Dialysemaschine
- 2: Internfluidik
- 3: Blutbehandlungseinheit, Dialysator
- 4: erste frischstromseitige Bilanzierungskammer
- 5: zweite frischstromseitige Bilanzierungskammer
- 6: erste brauchstromseitige Bilanzierungskammer
- 7: zweite brauchstromseitige Bilanzierungskammer
- 8: Zuflussleitung
- 9: erste Frischstrompumpe
- 10: erster Frischstromleitungsstrang
- 11: zweiter Frischstromleitungsstrang
- 12: Ventil
- 13: Ventil
- 14: Ventil
- 15: Ventil
- 16: Frischstromleitung
- 17: zweite Frischstrompumpe
- 18: Brauchstromleitung
- 19: erste Brauchstrompumpe
- 20: erster Brauchstromleitungsstrang
- 21: zweiter Brauchstromleitungsstrang
- 22: Ventil
- 23: Ventil
- 24: Ventil
- 25: Ventil
- 26: Brauchstromleitung
- 27: zweite Brauchstrompumpe
- 28: Abfluss
- 29: extrakorporale Blutleitung
- 30: Blutflussrichtung
- 31: Lüftungsöffnung
- 32: Messeinrichtung, Waage, Kraftsensor
- 33: Engstelle, Drossel
- 34: Ventil
- 35: Linearmotor
- 36: Linearmotor
- 37: Linearmotor
- 38: Linearmotor
- 39: Kolben
- 40: Kolben
- 41: Kolben
- 42: Kolben
- 43: frischstromseitige Bilanzierungskammer
- 44: brauchstromseitige Bilanzierungskammer
- 45: Ventil

## Patentansprüche

1. Vorrichtung (1) zur extrakorporalen Blutbehandlung, insbesondere Dialysemaschine (1), mit einer Internfluidik (2), an die eine Blutbehandlungseinheit (3), insbesondere ein Dialysator (3), anschließbar ist, wobei die Internfluidik (2) zumindest eine frischstromseitige Bilanzierungskammer (4, 5) zur Bilanzierung von zur Blutbehandlungseinheit (3) fließender Frischbehandlungsflüssigkeit und zumindest eine brauchstromseitige Bilanzierungskammer (6, 7) zur Bilanzierung von von der Blutbehandlungseinheit (3) abströmender Brauchbehandlungsflüssigkeit umfasst,
**dadurch gekennzeichnet, dass**
frischstromseitig und/oder brauchstromseitig der Behandlungseinheit (3) jeweils zwei Bilanzierungskammern (4, 5, 6, 7) angeordnet sind und die Vorrichtung (1) jeweils eine Messeinrichtung (32) zur gravimetrischen Erfassung von Behandlungsflüssigkeit in der frischstromseitigen Bilanzierungskammer (4, 5) und/oder jeweils eine Messeinrichtung (32) zur gravimetrischen Erfassung von Behandlungsflüssigkeit in der brauchstromseitigen Bilanzierungskammer (6, 7) aufweist.

2. Vorrichtung (1) nach Anspruch 1, **dadurch gekennzeichnet, dass** die Messeinrichtung (32) eine Waage und/oder einen Kraftsensor umfasst, insbesondere zur direkten Bestimmung der Masse von in der Bilanzierungskammer (4, 5, 6, 7) befindlicher Behandlungsflüssigkeit.

3. Vorrichtung (1) nach Anspruch 1 oder 2, **dadurch gekennzeichnet, dass** frischstromseitig und/oder brauchstromseitig der Behandlungseinheit (3) jeweils zwei Bilanzierungskammern (4, 5, 6, 7) angeordnet sind, die strömungstechnisch zueinander parallel angeordnet sind.

4. Vorrichtung (1) nach Anspruch 1 oder 2, **dadurch gekennzeichnet, dass** frischstromseitig und/oder brauchstromseitig der Behandlungseinheit (3) jeweils zwei Bilanzierungskammern (4, 5, 6, 7) angeordnet sind, die strömungstechnisch zueinander in Serie angeordnet sind.

5. Vorrichtung (1) nach einem der vorstehenden Ansprüche, **dadurch gekennzeichnet, dass** in der Internfluidik (2) zustromseitig und/oder abstromseitig der jeweiligen Bilanzierungskammer (4, 5, 6, 7) jeweils ein schaltbares Absperrventil (12, 13, 14, 15, 22, 23, 24, 25, 34, 45) angeordnet ist, zum Steuern / Regeln der Zu- bzw. Abströme von Behandlungsflüssigkeit zu oder aus der jeweiligen Bilanzierungskammer (4, 5, 6, 7).

6. Vorrichtung (1) nach einem der vorstehenden Ansprüche, **dadurch gekennzeichnet, dass** zumindest eine der Bilanzierungskammern (4, 5, 6, 7) eine Lüftungsöffnung (31) aufweist, die insbesondere mit einer Filtereinheit, insbesondere mit einem Hydrophobfilter und/oder einem Überdruckventil versehen ist.

7. Vorrichtung (1) nach einem der vorstehenden Ansprüche, **dadurch gekennzeichnet, dass** die Internfluidik (2) zustromseitig der Bilanzierungskammer (4, 5, 6, 7) und abstromseitig der Bilanzierungskammer (4, 5, 6, 7) jeweils eine Pumpe (9, 17, 19, 27) zur Förderung von Behandlungsflüssigkeit aufweist.

8. Vorrichtung (1) nach einem der Ansprüche 1 bis 6, **dadurch gekennzeichnet, dass** die Bilanzierungskammer (4, 5, 6, 7) als Kolben-Zylinder-Einheit mit der Bilanzierungskammer (4, 5, 6, 7) als Zylinder und einem mit dieser zusammenwirkenden und darin aufgenommenen Kolben (39, 40, 41, 42) ausgebildet ist.

9. Vorrichtung (1) nach einem der vorstehenden Ansprüche, **dadurch gekennzeichnet, dass** in der Internfluidik (2) stromauf der frischstromseitigen Bilanzierungskammer (4, 5) eine Engstelle (33) im Strömungsquerschnitt ausgebildet ist, zur Erzeugung eines lokalen Unterdrucks und Entgasung der Behandlungsflüssigkeit.

## Claims

1. A device (1) for extracorporeal blood treatment, in particular a dialysis machine (1), comprising an internal fluidic system (2) to which a blood treatment unit (3), in particular a dialyzer (3), can be connected, wherein the internal fluidic system (2) comprises at least one balancing chamber (4, 5) on the fresh flow side for balancing fresh treatment fluid flowing to the blood treatment unit (3) and at least one balancing chamber (6, 7) on the used flow side for balancing used treatment fluid flowing off the blood treatment unit (3),
**characterized in that**
two respective balancing chambers (4, 5, 6, 7) are arranged on the fresh flow side and/or on the used flow side of the treatment unit (3) and the device (1) has a respective measuring device (32) for gravimetric detection of treatment fluid in the balancing chamber (4, 5) on the fresh flow side and/or a measuring device (32) for gravimetric detection of treatment fluid in the balancing chamber (6, 7) on the used flow side.

2. The device (1) according to claim 1, **characterized in that** the measuring device (32) comprises a balance and/or a force sensor, in particular for the direct determination of the mass of treatment fluid present in the balancing chamber (4, 5, 6, 7).

3. The device (1) according to claim 1 or 2, **characterized in that** two balancing chambers (4, 5, 6, 7) are arranged in each case on the fresh flow side and/or used flow side of the treatment unit (3), said chambers being arranged parallel to one another in terms of fluidics.

4. The device (1) according to claim 1 or 2, **characterized in that** two balancing chambers (4, 5, 6, 7) are arranged in each case on the fresh flow side and/or used flow side of the treatment unit (3), said chambers being arranged in series with respect to one another in terms of fluidics

5. The device (1) according to one of the preceding claims, **characterized in that** a switchable shut-off valve (12, 13, 14, 15, 22, 23, 24, 25, 34, 45) is arranged in the internal fluidic system (2) in each case on the inflow side and/or outflow side of the respective balancing chamber (4, 5, 6, 7), for controlling/regulating the inflow and outflow of treatment fluid to or from the respective balancing chamber (4, 5, 6, 7).

6. The device (1) according to one of the preceding claims, **characterized in that** at least one of the balancing chambers (4, 5, 6, 7) has a ventilation opening (31) which is provided in particular with a filter unit, in particular with a hydrophobic filter and/or a pressure relief valve.

7. The device (1) according to one of the preceding claims, **characterized in that** the internal fluidic system (2) comprises a pump (9, 17, 19, 27) for pumping treatment fluid on the inflow side of the balancing chamber (4, 5, 6, 7) and on the outflow side of the balancing chamber (4, 5, 6, 7).

8. The device (1) according to one of claims 1 to 6, **characterized in that** the balancing chamber (4, 5, 6, 7) is designed as a piston-cylinder unit with the balancing chamber (4, 5, 6, 7) as a cylinder and a piston (39, 40, 41, 42) cooperating with it and accommodated therein.

9. The device (1) according to one of the preceding claims, **characterized in that** a constriction (33) in the flow cross-section is formed in the internal fluidic system (2) upstream of the balancing chamber (4, 5) on the fresh flow side, for generating a local negative pressure and degassing the treatment fluid.

## Revendications

1. Dispositif (1) pour le traitement extracorporel du sang, en particulier machine de dialyse (1), comportant un système fluidique interne (2) à laquelle une unité de traitement du sang (3), en particulier un dialyseur (3) peut être raccordé(e),
dans lequel le système fluidique interne (2) comprend au moins une chambre d'équilibrage du côté courant frais (4, 5) pour l'équilibrage du liquide de traitement frais s'écoulant vers l'unité de traitement du sang (3) et au moins une chambre d'équilibrage du côté courant utile (6, 7) pour l'équilibrage du liquide de traitement utile s'évacuant de l'unité de traitement du sang (3),
**caractérisé en ce que**
du côté courant frais et/ou courant utile de l'unité de traitement (3) sont disposées respectivement deux chambres d'équilibrage (4, 5, 6, 7) et le dispositif (1) présente respectivement un système de mesure (32) pour la saisie gravimétrique de liquide de traitement dans la chambre d'équilibrage du côté courant frais (4, 5) et/ou respectivement un système de mesure (32) pour la saisie gravimétrique de liquide de traitement dans la chambre d'équilibrage du côté courant utile (6, 7).

2. Dispositif (1) selon la revendication 1, **caractérisé en ce que** le système de mesure (32) comprend une balance et/ou un capteur de force, en particulier pour la détermination directe de la masse du liquide de traitement se trouvant dans la chambre d'équilibrage (4, 5, 6, 7).

3. Dispositif (1) selon la revendication 1 ou 2, **caractérisé en ce que** du côté courant frais et/ou du côté courant utile de l'unité de traitement (3) sont disposées respectivement deux chambres d'équilibrage (4, 5, 6, 7) qui sont disposées parallèlement l'une à l'autre d'un point de vue de la mécanique des fluides.

4. Dispositif (1) selon la revendication 1 ou 2, **caractérisé en ce que** du côté courant frais et/ou du côté courant utile de l'unité de traitement (3) sont disposées respectivement deux chambres d'équilibrage (4, 5, 6, 7) qui sont disposée en série l'une par rapport à l'autre d'un point de vue de la mécanique des fluides.

5. Dispositif (1) selon l'une quelconque des revendications précédentes, **caractérisé en ce que** dans le système fluidique interne (2) est disposé en amont et/ou en aval de la chambre d'équilibrage respective (4, 5, 6, 7) respectivement une vanne anti-retour commutable (12, 13, 14, 15, 22, 23, 24, 25, 34, 45) pour la commande/régulation des courants entrant ou sortant du liquide de traitement vers ou hors de la chambre d'équilibrage respective (4, 5, 6, 7).

6. Dispositif (1) selon l'une quelconque des revendications précédentes, **caractérisé en ce qu'**au moins l'une des chambres d'équilibrage (4, 5, 6, 7) présente une ouverture d'aération (31) qui est dotée en particulier d'une unité de filtrage, en particulier d'un filtre hydrophobe et/ou d'une vanne de surpression.

7. Dispositif (1) selon l'une quelconque des revendications précédentes, **caractérisé en ce que** le système fluidique interne (2) présente en amont de la chambre d'équilibrage (4, 5, 6, 7) et en aval de la chambre d'équilibrage (4, 5, 6, 7) respectivement une pompe (9, 17, 19, 27) pour l'acheminement du liquide de traitement.

8. Dispositif (1) selon l'une quelconque des revendications 1 à 6, **caractérisé en ce que** la chambre d'équilibrage (4, 5, 6, 7) est formée comme une unité piston-cylindre, la chambre d'équilibrage (4, 5, 6, 7) étant le cylindre et un piston (39, 40, 41, 42) coopérant avec celle-ci et étant reçu dans celle-ci.

9. Dispositif (1) selon l'une quelconque des revendications précédentes, **caractérisé en ce que** dans le système fluidique interne (2) en amont de la chambre d'équilibrage du côté du courant frais (4, 5), un rétrécissement (33) est formé dans la section d'écoulement, pour générer une pression négative locale et dégazer le liquide de traitement.
